# EUROPEAN PATENT SPECIFICATION

(11) **EP 0 560 532 B1**
(45) Date of publication and mention of the grant of the patent: **04.08.1999**
(21) Application number: 93301607.3
(22) Date of filing: 03.03.1993
(51) Int. Cl.: C07B 35/02, C07C 11/02, C07C 15/44, C07C 33/025, C07C 57/03, C07C 69/145, C07C 69/533, C07D 309/12, C07F 7/18, C07C 5/09, C07C 29/17, C07C 51/36, C07C 67/283, C07C 67/303

(54) **Preparation of cis-olefins**
Herstellung von Cis-Olefinen
Préparation de cis-oléfines

(30) Priority: 09.03.1992 JP 8595292
(43) Date of publication of application: 15.09.1993
(73) Proprietor: NISSAN CHEMICAL INDUSTRIES LTD., Chiyoda-ku Tokyo (JP); Sato, Fumie, Fujisawa-shi, Kanagawa-ken 251 (JP)
(72) Inventor: Sato, Fumie, Fujisawa-shi, Kanagawa-ken (JP); Miyaji, Kautsuaki, c/o Chuo Kenkyujo, Funabashi-shi, Chiba-ken (JP); Amano, Takehiro, c/o Taisho Parmaceutical Co. Ltd., Toshima-ku, Tokyo (JP)
(74) Representative: Stoner, Gerard Patrick

(56) References cited:
- JOURNAL OF ORGANIC CHEMISTRY. vol. 45, no. 24, 21 November 1980, EASTON US pages 4926 - 4931 J. R. WEIR ET AL 'PALLADIUM-CATALYZED TRIETHYLAMMONIUM FORMATE REDUCTIONS. 4. REDUCTION OF ACETYLENES TO CIS MONOENES AND HYDROGENOLYSIS OF TERTIARY ALLYLIC AMINES'
- JOURNAL OF ORGANIC CHEMISTRY. vol. 43, no. 20, 29 September 1978, EASTON US pages 3985 - 3987 N. A. CORTESE ET AL 'PALLADIUM-CATLYZED REDUCTIONS OF ALPHA,BETA-UNSATURATED CARBONYL COMPOUNDS, CONJUGATED DIENES, AND ACETYLENES WITH TRIALKYLAMMONIUM FORMATES'
- TETRAHEDRON, (INCL. TETRAHEDRON REPORTS) vol. 44, no. 2, 1988, OXFORD GB pages 481 - 490 A. ARCADI ET AL 'THE PALLADIUM-TRIBUTYLAMMONIUM FORMATE REAGENT IN THE STEREOSELECTIVE HYDROGENATION, AND STEREO- AND REGIOSELECTIVE HYDROARYLATION OF ALKYL 4-HYDROXY-2-ALKYNOATES: A ROUTE TO SUBSTITUTED BUTENOLIDES'

## Description

This invention relates to the preparation of cis-olefins, and more particularly, a method for preparing cis-olefins through formic acid reduction of alkynes in the presence of palladium catalysts.

Cis-olefins are useful intermediates for the synthesis of many fine chemicals, especially for the synthesis of bioactive materials having a double bond cis-conformed in their structural formula.

In the prior art, cis-olefins were prepared by the hydrogen reduction of alkynes using Lindlar catalysts. Also known in the art is silicon hydride reduction using palladium catalysts (Barley M. Trost, Tetrahedron Letters, Vol. 30, No. 35 pp. 4657, 1989).

These method have several drawbacks. In the hydrogen reduction method using Lindlar catalysts it is difficult to obtain cis-olefins of high purity since trans-olefins and saturated compounds are by-produced in addition to the desired cis-olefins. The use of hydrogen creates a fire hazard. The silicon hydride reduction method must use expensive silicon hydrides and is generally low in cis-olefin selectivity so that trans-olefins can be a major product.

In Tetrahedron, Vol. 44, pp. 481, 1988, A. Arcadi et al disclose formic acid reduction in which aryl iodides are reacted with alkyl 4 - hydroxy - 2 - alkynoates and in the presence of formic acid, tri-n-butylamine and a palladium ( 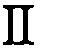 ) catalyst.

However, the above formic acid reduction method only provides cyclic product as shown below. No cis-olefins which are pure hydrogenated products are obtainable.

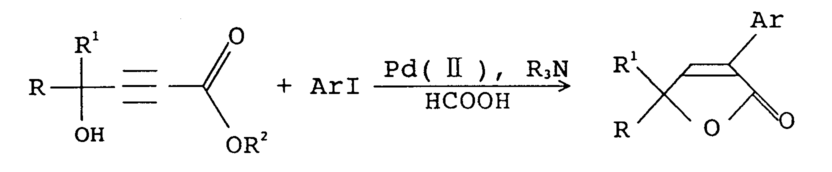

Arcadi et al. also disclose the reductive cyclisation of alkyl 4-hydroxy-2-alkanoates by treatment with formic acid in the presence of a palladium catalyst and tri-n-butylamine.

In J. Org. Chem., Vol. 45, No. 24, pp4926-4931, 1980, J.R. Weir et al. disclose the reduction by formic acid of a variety of alkynes having a substituted or unsubstituted aliphatic hydrocarbon group or a substituted or unsubstituted phenyl group in the presence of a Pd catalyst and triethylamine to produce cis-olefins. In J. Org. Chem., Vol. 43, No. 20, pp3985-3987, 1978, N.A. Cortese and R.F. Heck disclosed a range of reductions of α,β-unsaturated carbonyls, conjugated dienes and acetylenes using trialkylammonium formates. Most were at 100°C with Pd-on-carbon catalyst, 95% formic acid and trimethylamine or tri-n-butylamine. However one reduction of 3-hexyne used palladium diacetate catalyst with also tri-o-tolylphosphine, at room temperature, to give cis-3-hexene at 85% yield.

There is a need to have a simple method capable of selectively producing cis-olefins in high yields.

The present invention provides a method for preparing a cis-olefin of the following general formula (2): 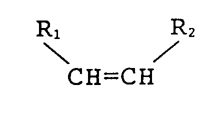 wherein R₁ and R₂ are independently selected from ester group, substituted silyl group, carboxyl group, cyano group, substituted and unsubstituted aliphatic hydrocarbon group having 1 to 20 carbon atoms and substituted and unsubstituted phenyl group, comprising the step of reducing an alkyne of the following general formula (1):

R₁ - C ≡ C - R₂ (1)

wherein R₁ and R₂ are defined above, with formic acid in the presence of a palladium catalyst, amine, and a phosphine or phosphite, the amine being added in an amount of 0.1 to 10 equivalent per equivalent of the alkyne and the phosphine or phosphite being added in an amount of 0.1 to 100 mol% based on moles of the alkyne, the reduction being carried out in solvent selected from ether, hydrocarbon, ester, amide and sulphoxide solvents and mixtures thereof.

### DETAILED DESCRIPTION

The method starts with an alkyne of formula (1).

R₁ - C ≡ C - R₂ (1)

In formula (1), R₁ and R₂, which may be identical or different, are independently selected from the group consisting of (a) an ester group, (b) a substituted silyl group, (c) a carboxyl group, (d) a cyano group, (e) a substituted or unsubstituted aliphatic hydrocarbon group having 1 to 20 carbon atoms, and (f) a substituted or unsubstituted phenyl group.
Examples of the group represented by R₁ and R₂ are given below.

Examples of (a) ester group include substituted or unsubstituted alkoxycarbonyl groups having 1 to 10 carbon atoms and substituted or unsubstituted phenoxycarbonyl groups. The substituted or unsubstituted alkoxycarbonyl groups having 1 to 10 carbon atoms include methoxycarbonyl, ethoxycarbonyl, n-propoxycarbonyl, i-propoxycarbonyl, n-butoxycarbonyl, sec-butoxycarbonyl, t-butoxycarbonyl, pentoxycarbonyl, hexoxycarbonyl, heptoxycarbonyl, octoxycarbonyl, nonanoxycarbonyl, decisoxycarbonyl, methoxymethoxycarbonyl, methylthiomethoxycarbonyl, tetrahydropyranoxycarbonyl, tetrahydrofuranotoxycarbonyl, benzyloxymethoxycarbonyl, phenasiloxycarbonyl, o-methylphenoxymethoxycarbonyl, m-methylphenoxymethoxycarbonyl, p-methylphenoxymethoxycarbonyl, o - bromophenasiloxycarbonyl, m-bromophenasiloxycarbonyl, p - bromophenasiloxycarbonyl, benzyloxycarbonyl, o-methylphenasiloxycarbonyl, m-methylphenasiloxycarbonyl, p-methylphenasiloxycarbonyl, 2,2,2 - trichloroethoxycarbonyl, 2 - chloroethoxycarbonyl, 2-methylthioethoxycarbonyl, cyclopentyloxycarbonyl, cyclohexyloxycarbonyl, allyloxycarbonyl groups, etc. The substituted or unsubstituted phenoxycarbonyl groups include biphenoxycarbonyl, 4 - (4 - fluorophenyl)phenoxycarbonyl, 4 - (4 - chlorophenyl)phenoxycarbonyl, 4 - (4 - bromophenyl)phenoxycarbonyl, 4 - (4 - iodophenyl) - phenoxycarbonyl groups.

Examples of (b) substituted silyl group include trimethylsilyl, i-propyldimethylsilyl, t-butyldimethylsilyl groups.

Examples of (e) unsubstituted aliphatic hydrocarbon group having 1 to 20 carbon atoms include methyl, ethyl, n-propyl, i-propyl, n-butyl, i-butyl, sec-butyl, t-butyl, n-amyl, i-amyl, hexyl, heptyl, octyl, nonyl, decyl, undecyl, dodecyl, tridecyl, hexadecyl, octadecyl, eicosyl groups.

The substituents on (e) aliphatic hydrocarbon groups and (f) phenyl group include hydroxyl, protected hydroxyl, halogen, formyl, ester, carbonyl, amide, carboxyl, cyano, p-methylthio, phenyl, fluorophenyl, chlorophenyl, bromophenyl, and iodophenyl groups.

By the protected hydroxyl groups are meant those hydroxyl groups protected with substituted silyl groups such as trimethylsilyl and t-butyldimethylsilyl groups, alkoxyalkyl groups such as methoxymethyl and ethoxymethyl groups, acyl groups such as acetyl and benzoyl groups, trityl, tetrahydropyranyl, benzyl and p-chlorobenzyl groups.

The ester substituents include substituted or unsubstituted alkoxycarbonyl groups having 1 to 10 carbon atoms and substituted or unsubstituted phenoxycarbonyl groups as mentioned for (a).

The halogen substituents include fluorine, chlorine, bromine and iodine atoms.

The amide substituents include substituted or unsubstituted alkoxycarbonylamide groups having 1 to 10 carbon atoms and substituted or unsubstituted phenoxycarbonylamide groups. The substituted or unsubstituted alkoxycarbonylamide groups having 1 to 10 carbon atoms include methoxycarbonylamino, ethoxycarbonylamino, n-propoxycarbonylamino, i-propoxycarbonylamino, n-butoxycarbonylamino, sec-butoxycarbonylamino, t-butoxycarbonylamino, pentoxycarbonylamino, hexoxycarbonylamino, heptoxycarbonylamino, octoxycarbonylamino, nonanoxycarbonylamino, decisoxycarbonylamino, methoxymethoxycarbonylamino, methylthiomethoxycarbonylamino, tetrahydropyranoxycarbonylamino, tetrahydrofuranotoxycarbonylamino, benzyloxymethoxycarbonylamino, phenasiloxycarbonylamino, o-methylphenoxymethoxycarbonylamino, m-methylphenoxymethoxycarbonylamino, p-methylphenoxymethoxycarbonylamino, o-bromophenasiloxycarbonylamino, m-bromophenasiloxycarbonylamino, p-bromophenasiloxycarbonylamino, benzyloxycarbonylamino, o-methylphenasiloxycarbonylamino, m-methylphenasiloxycarbonylamino, p-methylphenasiloxycarbonylamino, 2,2,2-trichloroethoxycarbonylamino, 2-chloroethoxycarbonylamino, 2-methylthioethoxycarbonylamino, cyclopentyloxycarbonylamino, cyclohexyloxycarbonylamino, allyloxycarbonylamino groups, etc. The amide moiety of the alkoxycarbonylamide and phenoxycarbonylamide groups may have a substituent which is selected from the above-mentioned substituents on the C1 - C10 aliphatic hydrocarbon and phenyl groups and which may further have a substituent such as a hydroxyl, protected hydroxyl, halogen, formyl, ester, amide, carboxyl, cyano, p-methylthio, phenyl, fluorophenyl, chlorophenyl, bromophenyl, and iodophenyl group.

Illustrative, non-limiting examples of the alkyne of formula (1) include 1-hydroxy-4-heptyne, 1-hydroxy-2-octyne, 3-hydroxy-4-decyne, 2-hydroxy-2-methyl-3-nonyne, 1-trimethylsilyl-1-heptyne, 1-t-butyldimethylsiloxy-2-octyne, 1-trimethylsilyl-3-hydroxy-1-octyne, 1-carboxy-3-hexyne, 1-carboxy-1-heptyne, 1-methoxycarbonyl-1-heptyne, 1-ethoxycarbonyl-4-heptyne, 1-allyloxy-carbonyl-1-heptyne, 1-methoxycarbonyl-6-hydroxy-4-hexyne, 1-ethoxycarbonyl-3-hexyne, 3-oxy-4-decyne, 2-oxy-5-octyne, 1-benzyloxy-2-octyne, 1-tetrahydropyranoxy-2-octyne, 1-formyl-3-hexyne, 1-bromo-4-heptyne, 1-cyano-4-heptyne, 1-phenyl-1-butyne, and 1-phenylthio-4-heptyne.

According to the present invention, these alkynes are reduced with formic acid in the presence of palladium catalyst. The palladium catalysts used herein may be complexes of palladium having a valence of zero or plus two.

Examples of the palladium catalyst include Pd(PPh₃)₄, Pd(Ph^{t}Bu₂)₂, Pd(P^{t}Bu₃)₂, Pd[P(c-C₆H₁₁)₃]₂, Pd[P(OPh)₃]₄, Pd[P(OEt)₃]₄, Pd(AsPh₃)₄, Pd(CO)(PPh₃)₃, Pd(PPh₃)₂(CH₂=CH₂), Pd(PPh₃)₂(CF₂=CF₂), Pd[P(c-C₆H₁₁)₃]₂(CH₂=CH₂), Pd[P(c-C₆H₁₁)₃]₂(CF₂=CF₂), Pd(PBu₃)₂(CH₂=CH₂), Pd(PPh₃)₂(CF₂=CF₂), Pd[P(OC₆H₄CH₃-o)]₂(CH₂=CH₂), Pd(PPh₃)₂(CH₃OOCCH=CHCOOCH₃), Pd[P(OPh)₃]₂(CH₃OOCCH=CHCOOCH₃), Pd(PPh₃)₂(NCCH=CHCN), Pd[P(OPh)₃]₂(NCCH=CHCN), Pd(PPh₂Me)₂(PhCH=CH₂), Pd(PPh₂Me)₂(CH₂=CHCOOMe), Pd(PPh₂Me)₂[CH₂=CH(CH₃)CN], Pd(PPh₂Me)₂[CH₂=CH(CH₃)COMe], Pd(1,5-C₈H₁₂)₂, Pd₂(DBA)₃, Pd₃(CO)₃(P^{t}Bu₃)₃, Pd₃(2,6-Me₂C₆H₃NC)₆, PdCl(CH₃)(COD), Pd(CH₃)₂(PEt₃)₂, Pd(C₂H₅)₂(PMe₂Ph)₂, Pd(CH₃)₂(DPPE), Pd(CH₃)(PPh₃)₂, PdPh₂(PEt₃)₂, Pd(CH₂Ph)₂(DMPE), Pd(CH₂Ph)₂(PMe₃)₂, Pd(CH₃)Cl(PEt₃)₂, Pd(CH₃)I(PMe₃)₂, Pd(CH₃)Br(PEt₃)₂, PdI(Ph)(PMe₃)₂, Pd(COPh)Cl(PMe₃)₂, Pd (COMe)I(PMe₃Ph₂)₂, Pd (COMe)Cl(PPh₃)₂, Pd(COEt)Cl[P(OMe₃) ]₂, PdI(Ph)(PMe₂Ph)₂, Pd(CH ≡ CH)₂ (PEt₃)₂, Pd(PhC ≡ C)Cl(PBu₃)₂, Pd(Me₃CCH₂)₂(BPY), Pd(C₅H₆)Ph(PEt₃), Pd( η-C₃H₅)(C₅Me₅), [Pd( η-C₃H₅)(COD)]BF₄, [PdCl(1,1-Me₂-C₃H₅)]₂, Pd₂(DBA)₃CHCl₃, Pd(DBA)₂, PdCl₂(MeCN)₂, PdCl₂(PhCN)₂, Li₂PdCl₄, Na₂PdCl₄, [Pd(MeCN)₄](BF₄)₂, [Pd( η-C₃H₅)Cl]₂, Pd( η-C₃H₅)₂ and Pd(OAc)₂.

Note that DBA is dibenzylideneacetone, COD is cyclooctadiene, DPPE is diphenylphosphin-ethane, DMPE is dimethylphosphinethane, and BPY is dipyridyl.

The palladium catalyst may be used in an amount of about 0.1 to 100 moles, preferably about 1 to 10 mol% based on the moles of the alkyne of formula (1).

Formic acid may be used in an amount of about 1 to 1000 mol%, preferably 10 to 500 mol% based on the moles of the alkyne of formula (1).

To the reaction system is added an amine and phosphine or phosphite.

The amines usable herein are of the following general formulae (3) to (5), the phosphines are of the following general formula (6), and the phosphites are of the following general formula (7).

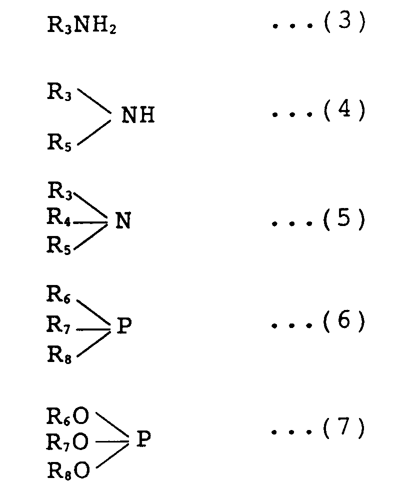

In these formulae, R₃, R₄, R₅, R₆, R₇, and R₈ are independently hydrocarbon groups having 1 to 10 carbon atoms, for example, methyl, ethyl, n-propyl, i-propyl, n-butyl, i-butyl, sec-butyl, t-butyl, n-amyl, i-amyl, hexyl, heptyl, octyl, nonyl, decyl, benzyl, and phenyl groups.

The amines are added in amounts of 0.1 to 10 equivalents, more preferably 0.5 to 5 equivalents per equivalent of the alkyne of formula (1). Also the phosphines or phosphites are added in amounts of 0.1 to 100 mol%, more preferably 1 to 30 mol% based on the moles of the alkyne of formula (1).

In the practice of this method, reduction may be carried out in a suitable solvent, such as ether, hydrocarbon, ester, amide, and sulfoxide solvents. Suitable ether solvents are diethyl ether, di-n-propyl ether, di-i-propyl ether, tetrahydrofuran, and 1,4-dioxane. Suitable hydrocarbon solvents are n-pentane, i-pentane, n-hexane, benzene, toluene, and xylene. Suitable ester solvents are methyl acetate, ethyl acetate, n-propyl acetate, and i-propyl acetate. Suitable amide solvents are dimethylformamide and dimethyl-acetamide. A suitable sulfoxide solvent is dimethylsulfoxide. These solvents may be used alone or in admixture of two or more.

The reducing temperature generally ranges from about -40°C to the reflux temperature of the solvent, preferably from about - 10°C to about 60°C. The reaction time may be from about 1/2 to 12 hours.

By reducing alkynes of formula (1) with formic acid in the presence of a palladium catalyst as described, we have been able to make cis-olefins of formula (2) selectively, in high yields, with scarcely any formation of trans-olefins and saturated compounds. 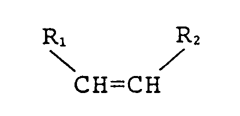 In formula (2), R₁ and R₂ are as defined above.

Furthermore, we found that the method could be carried out under moderate conditions, which is practically advantageous. The resulting cis-olefins are useful compounds, e.g. as intermediates for the synthesis of fine chemicals.

### EXAMPLE

Examples of the present invention are given below by way of illustration and not by way of limitation.

### Example 1

In 50 ml of tetrahydrofuran was dissolved 20.9 ml (150 mmol) of triethylamine. To the solution were added 6.38 grams (122 mmol) of formic acid in water (concentration 88% by weight), 1.29 grams (1.25 mmol) of Pd₂(DBA)₃·CHCl₃, and 1.25 ml (5 mmol) of n-tributylphosphine. The mixture was agitated for 10 minutes at room temperature. To the solution was added dropwise a solution containing 12.0 grams (79 mmol) of 2-undecyne in 150 ml of tetrahydrofuran. The mixture was agitated for two hours at 40°C.

At the end of reaction, 50 ml of a saturated ammonium chloride aqueous solution and 100 ml of n-hexane were added to the reaction solution. The organic layer was extracted, dried over magnesium sulfate, and filtered. By distilling off the solvent, there was obtained 12 grams of 2-undecene in a substantially quantitative yield.

The result of gas chromatography revealed that the cis-undecene was at least 99% pure and formation of trans-undecene and undecane was less than 1% for each.

The analytical results of the product are shown below.
H NMR (CDCl₃, 300MHz)
   - δ: 0.87 (t, J = 5.9 Hz, 3 H).
   1.20~1.40 (m, 12H).
   1.60 (d, 1=5.0Hz, 3H).
   1.95~2.08 (m, 2H).
   5.32~5.50 (m, 2H)
¹³C NMR (CDCl₃, 75MHz)
   - δ: 130.9, 123.6, 31.9, 29.7, 29.6, 29.3, 26.9, 22.7, 14.1

### Examples 2-11

The procedure of Example 1 was repeated except that the starting alkyne and the reaction time were changed as shown in Table 1. The reaction scheme is shown below.

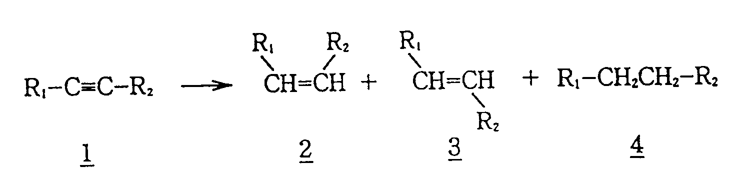

**Table 1**

| Example | Alkyne (1) | Reaction time, hr. | Selectivity, % | | | Yield (%) |
|---|---|---|---|---|---|---|
| | | | (2) | (3) | (4) | |
| 2 | 1-methoxycarbonyl-1-heptyne | 2 | 98 | 2 | 0 | 95 |
| 3 | 1-phenyl-1-butyne | 3 | 89 | 5 | 6 | 100 |
| 4 | 1-hydroxy-2-octyne | 1.5 | 99 | 0 | 1 | 91 |
| 5 | 3-hydroxy-4-decyne | 3 | > 99 | < 1 | < 1 | 90 |
| 6 | 1-carboxy-1-heptyne | 3 | > 99 | < 1 | < 1 | 100 |
| 7 | 3-acetoxy-4-decyne | 3 | > 99 | < 1 | < 1 | 100 |
| 8 | 2-hydroxy-2-methyl-3-octyne | 3 | > 99 | < 1 | < 1 | 100 |
| 9 | 1-t-butyldimethylsilyloxy-2-octyne | 3 | > 99 | < 1 | < 1 | 100 |
| 10 | 1-tetrahydropyranoxy-2-octyne | 3 | > 99 | < 1 | < 1 | 100 |
| 11 | 1-ethoxycarbonyl-3-hexyne | 3 | > 99 | < 1 | < 1 | 100 |

The analytical data of products (2) are shown below.

### Example 2

H NMR (CDl₃, 300MHz)
   - δ: 0.89 (t, J = 6.5 Hz, 3 H),
   1.21∼1.62 (m, 6H),
   2.65 (dt, J =7.4, 7.2Hz, 2H),
   3.71 (s, 3H),
   5.77 (d, J = 11.5Hz, 1H),
   6.24 (dt, J = 11.5, 7.5Hz, 1H)

### Example 3

H NMR (CCl₄, 90 MHz)
   - δ: 1.00 (t, J = 7.9 Hz, 3H),
   2.25∼2.65 (m, 2H),
   5.48 (dt, J=7.9, 12.5Hz, 1H),
   6.25 (d, J=12.5Hz, 1H),
   7.00∼7.25 (m, 5H)

### Example 4

H NMR (CDl₃, 300MHz)
   - δ: 0.89 (t, J = 6.6Hz, 3H),
   1.15∼1.50 (m, 6H),
   1.88 (brs, 1H),
   2.06 (dt, J=6.9, 6.9Hz, 2H),
   4.19 (d, J=5.8 Hz, 2H),
   5.47∼5.70 (m, 2H)
¹³C NMR (CDCl₃, 75MHz)
   - δ: 133.0, 128.3, 58.4, 31.3, 29.2, 27.3, 22.5, 14.0

### Example 5

H NMR (CDCl₃, 300MHz)
   - δ: 0.78∼1.00 (m, 6H),
   1.20∼1.70 (m, 9H),
   1.95∼2.18 (m, 2H),
   4.25∼4.42 (m, 1H),
   5.27∼5.40 (m, 1H),
   5.51 (dt, J=7.3, 10.9Hz, 1H),
¹³C NMR (CDCl₃, 75MHz)
   - δ: 132.6, 132.2, 69.0, 31.4. 30.3, 29.4, 27.7, 22.5, 14.0, 9.7

### Example 6

H NMR (CDCl₃, 300MHz)
   - δ: 0.89 (t. J = 6.1Hz, 3H),
   1.20∼1.60 (m, 6H),
   2.60∼2.71 (m, 2H),
   5.79 (d. J = 11.5Hz, 1H),
   6.36 (dt, J = 7.5, 11.5 Hz, 1H)

### Example 7

H NMR (CDCl₃, 300MHz)
   - δ: 0.75~1.00 (m, 6H),
   1.18∼1.43 (m, 6H),
   1.43∼1.73 (m, 2H),
   2.03 (s, 3H),
   2.05∼2.22 (m, 2H),
   5.22∼5.34 (m, 1H),
   5.40∼5.61 (m, 2H)
¹³C NMR (CDCl₃, 75MHz)
   - δ: 170.4, 134.4, 127.7, 71.6, 31.4, 29.2, 27.8, 27.7, 22.5, 21.3, 14.0, 9.4

### Example 8

H NMR (CDCl₃, 300MHz)
   - δ: 0.89 (t, J=6.4Hz, 3H),
   1.37 (s, 6H),
   1.15∼1.65 (m, 6H),
   2.31 (d t, J=6.9, 6.5Hz, 2H),
   5.31 (dt, J=11.8, 7.3Hz, 1H),
   5.48 (d, J = 11.8Hz, 1H)
¹³C NMR (CDCl₃, 75MHz)
   - δ: 136.6, 131.4, 71.6, 31.6, 31.1, 29.7, 28.0, 22.6, 14.0

### Example 9

H NMR (CDCl₃, 300MHz)
   - δ: 0.12 (s, 6H),
   0.98 (s, 9H),
   0.80∼1.06 (m, 3H),
   1.20∼1.53 (m, 6H),
   1.93∼2.13 (m, 2H),
   4.13 (d. J=4.5Hz, 2H),
   5.13∼5.60 (m, 2H)

### Example 10

H NMR (CDCl₃, 300MHz)
   - δ: 0.88 (t, J=7.1Hz, 3H),
   1.20∼1.95 (m, 12H),
   2.08 (d t, J=7.1, 6.6Hz, 2H),
   3.45∼3.59 (m, 1H),
   3.82∼3.96 (m, 1H),
   4.07 and 4.26 (2 dd, J = 5.9, 12.2Hz and 4.9, 12.2Hz, 2H),
   4.64 (brs, 1H),
   5.50∼5.65 (m, 2H)
¹³C NMR (CDCl₃, 75MHz)
   - δ: 133.8, 125.6, 97.8, 62.7, 62.2, 31.4. 30.6, 29.2, 27.5, 25.4, 22.5, 19.5, 14.0

### Example 11

H NMR (CDCl₃, 90MHz)
   - δ: 0.93 (t, J=9.0Hz, 3H),
   1.20 (t, J=8.1Hz, 3H),
   1.82∼2. 30 (m, 6H),
   3.95 (q, J=8.1Hz, 2H),
   4.95∼5. 43 (m, 2H)

## Claims

1. A method for preparing a cis-olefin of the following general formula (2): 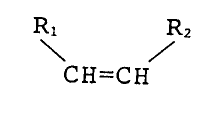 wherein R₁ and R₂ are independently selected from ester group, substituted silyl group, carboxyl group, cyano group, subsituted and unsubsituted aliphatic hydrocarbon group having 1 to 20 carbon atoms and substituted and unsubstituted phenyl group, comprising the step of reducing an alkyne of the following general formula (1):
R₁ - C ≡ C - R₂ (1)
wherein R₁ and R₂ are as defined above, with formic acid in the presence of a palladium catalyst, amine, and a phosphine or phosphite, the amine being added in an amount of 0.1 to 10 equivalents per equivalent of the alkyne and the phosphine or phosphite being added in an amount of 0.1 to 100 mol% based on moles of the alkyne, the reduction being carried out in solvent selected from ether, hydrocarbon, ester, amide and sulphoxide solvents and mixtures thereof.

2. A method according to claim 1 in which the solvent is selected from diethyl ether, di-n-propyl ether, di-i-propyl ether, tetrahydrofuran, 1, 4-dioxane, n-pentane, n-hexane, benzene, toluene, xylene, methyl acetate, ethyl acetate, n-propyl acetate, i-propyl acetate, dimethylformamide, dimethylacetamide and dimethylsulphoxide.

3. A method according to claim 1 or claim 2 where the palladium catalyst is used in an amount of 1 to 10 mol% based in the moles of the alkyne of formula (1), the formic acid is used in amount of 10 to 500 mol% based on the moles of the alkyne, the amine is added in an amount of 0.5 to 5 equivalents per equivalent of the alkyne, and the phosphine or phosphite is added in an amount of 1 to 30 mol% based on moles of the alkyne.

4. A method according to any one of the preceding claims where the ester group is substituted or unsubstituted alkoxycarbonyl group having 1 to 10 carbon atoms or a substituted or unsubstituted phenoxycarbonyl group.

5. A method according to any one of the preceding claims where the substituted silyl group is a trimethylsilyl, i-propyldimethylsilyl or t-butyldimethylsilyl group.

6. A method according to any one of the preceding claims, where the substituents on the aliphatic hydrocarbon group or on the phenyl group are chosen independently from hydroxyl, protected hydroxyl, halogen, formyl, ester, carbonyl, amide, carboxy, cyano, p-methylthio, phenyl, fluorophenyl, chlorophenyl, bromophenyl and iodophenyl groups.

7. A method according to any one of the preceding claims, where the alkyne of formula (1) is 1-methoxycarbonyl-1-heptyne, 1-phenyl-1-butyne, 1-hydroxy-2-octyne, 3-hydroxy-4-decyne, 1-carboxy-1-heptyne, 3-acetoxy-4-decyne, 2-hydroxy-2-methyl-3-octyne, 1-t-butyldimethylsiloxy-2-octyne, 1-tetrahydropyranoxy-2-octyne or 1-ethoxycarbonyl-3-hexyne.

8. A method according to any one of the preceding claims where the palladium catalyst is a complex of palladium with a valence of zero or plus two.

9. A method according to any one of claims 1 to 7 in which the palladium catalyst is selected from Pd(PPh₃)₄, Pd(Ph^{t}Bu₂)₂, Pd(P^{t}Bu₃)₂, Pd[P(c-C₆H₁₁)₃]₂, Pd[P(OPh)₃]₄, Pd[P(OEt)₃]₄, Pd(AsPh₃)₄, Pd(CO)(PPh₃)₃, Pd(PPh₃)₂(CH₂=CH₂), Pd(PPh₃)₂(CF₂=CF₂), Pd[P(c-C₆H₁₁)₃]₂(CH₂=CH₂), Pd[P(c-C₆H₁₁)₃]₂(CF₂=CF₂), Pd(PBu₃)₂(CH₂=CH₂), Pd(PPh₃)₂(CF₂=CF₂), Pd[P(OC₆H₄CH₃-o)]₂(CH₂=CH₂), Pd(PPh₃)₂(CH₃OOCCH=CHCOOCH₃), Pd[P(OPh)₃]₂(CH₃OOCCH=CHCOOCH₃), Pd(PPh₃)₂(NCCH=CHCN), Pd[P(OPh)₃]₂(NCCH=CHCN), Pd(PPh₂Me)₂(PhCH=CH₂), Pd(PPh₂Me)₂(CH₂=CHCOOMe), Pd(PPh₂Me)₂[CH₂=CH(CH₃)CN], Pd(PPh₂Me)₂[CH₂=CH(CH₃)COMe], Pd(1,5-C₈H₁₂)₂, Pd₂(DBA)₃, Pd₃(CO)₃(P^{t}Bu₃)₃, Pd₃(2,6-Me₂C₆H₃NC)₆, PdCl(CH₃)(COD), Pd(CH₃)₂(PEt₃)₂, Pd(C₂H₅)₂(PMe₂Ph)₂, Pd(CH₃)₂(DPPE), Pd(CH₃)(PPh₃)₂, PdPh₂(PEt₃)₂, Pd(CH₂Ph)₂(DMPE), Pd(CH₂Ph)₂(PMe₃)₂, Pd(CH₃)Cl(PEt₃)₂, Pd(CH₃)I(PMe₃)₂, Pd(CH₃)Br(PEt₃)₂, PdI(Ph)(PMe₃)₂, Pd(COPh)Cl(PMe₃)₂, Pd(COMe)I(PMe₃Ph₂)₂, Pd(COMe)Cl(PPh₃)₂, Pd(COEt)Cl[P(OMe₃)]₂, PdI(Ph)(PMe₂Ph)₂, Pd(CH≡CH)₂ (PEt₃)₂, Pd(PhC≡C)Cl(PBu₃)₂, Pd(Me₃CCH₂)₂(BPY), Pd(C₅H₆)Ph(PEt₃), Pd(η-C₃H₅)(C₅Me₅), [Pd(η-C₃H₅)(COD)]BF₄, [PdCl(1,1-Me₂-C₃H₅)]₂, Pd₂(DBA)₃CHCl₃, Pd(DBA)₂, PdCl₂(MeCN)₂, PdCl₂(PhCN)₂, Li₂PdCl₄, Na₂PdCl₄, [Pd(MeCN)₄](BF₄)₂, [Pd(η-C₃H₅)Cl]₂, Pd(η-C₃H₅)₂ and Pd(OAc)₂, wherein
DBA is dibenzylideneacetone.
COD is cyclooctadiene.
DPPE is diphenylphosphinethane.
DMPE is dimethylphosphinethane.
BPY is dipyridyl.

10. A method according to claim 9 in which the palladium catalyst is Pd₂(DBA)₃CHCl₃.

11. A method according to any one of the preceding claims where the amine is selected from those of the following general formulae (3) to (5); any phosphine is of the following general formula (6) and any phosphite is of the following general formula (7): 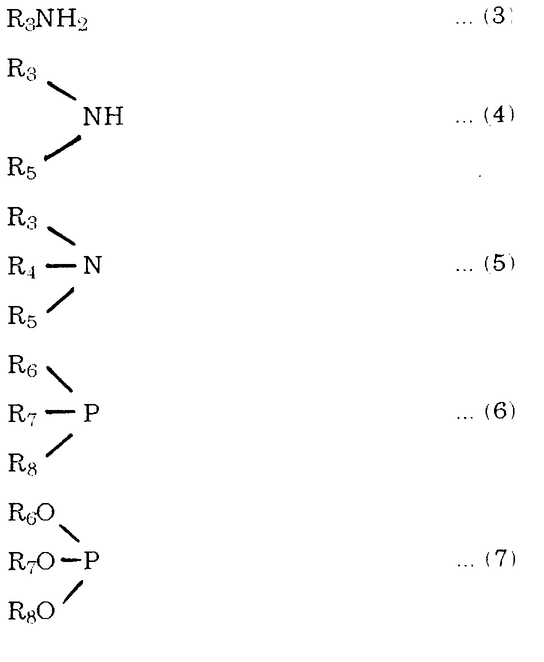 where R₃, R₄, R₅, R₆, R₇ and R₈ are independently selected from hydrocarbon groups having 1 to 10 carbon atoms.

12. A method according to claim 1 in which a phosphine is used and is n-tributylphosphine and the amine is triethylamine.

13. A method according to any one of the preceding claims where the reducing temperature lies in the range from -40°C to the reflux temperature of the solvent used and the reaction time is from ½ to 12 hours.

## Patentansprüche

1. Verfahren zur Herstellung einem cis-Olefins der folgenden allgemeinen Formel (2): 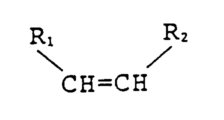 worin R₁ und R₂ unabhängig voneinander aus Ester-Gruppen, substituierten Silyl-Gruppen, Carboxyl-Gruppen, Cyano-Gruppen, substituierten und unsubstituierten aliphatischen Kohlenwasserstoff-Gruppen mit 1 bis 20 Kohlenstoffatomen und substituierten und unsubstituierten Phenyl-Gruppen ausgewählt sind,
umfassend den Schritt des Reduzierens eines Alkins der folgenden allgemeinen Formel (1):
R₁ - C ≡ C - R₂ (1)
worin R₁ und R₂ wie oben definiert sind, mit Ameisensäure in Gegenwart eines Palladiumkatalysators, Amins und eines Phosphins oder Phosphits, wobei das Amin in einer Menge von 0,1 bis 10 Äquivalenten pro Äquivalent des Alkins zugegeben wird und das Phosphin oder Phosphit in einer Menge von 0,1 bis 100 Mol-%, bezogen auf Mol des Alkins, zugegeben wird, wobei die Reduktion in einem Lösungsmittel durchgeführt wird, das aus Ether-, Kohlenwasserstoff-, Ester-, Amid- und Sulfoxid-Lösungsmitteln und Gemischen davon ausgewählt ist.

2. Verfahren nach Anspruch 1, bei dem das Lösungsmittel aus Diethylether, Di-n-propylether, Di-i-propylether, Tetrahydrofuran, 1,4-Dioxan, n-Pentan, n-Hexan, Benzol, Toluol, Xylol, Methylacetat, Ethylacetat, N-Propylacetat, i-Propylacetat, Dimethylformamid, Dimethylacetamid und Dimethylsulfoxid ausgewählt ist.

3. Verfahren nach Anspruch 1 oder 2, bei dem der Palladiumkatalysator in einer Menge von 1 bis 10 Mol-%, bezogen auf die Mol des Alkins der Formel (1), eingesetzt wird, die Ameisensäure in einer Menge von 10 bis 500 Mol-%, bezogen auf die Mol des Alkins, eingesetzt wird, das Amin in einer Menge von 0,5 bis 5 Äquivalenten pro Äquivalent des Alkins zugegeben wird und das Phosphin oder Phosphit in einer Menge von 1 bis 30 Mol-%, bezogen auf die Mol des Alkins, zugegeben wird.

4. Verfahren nach einem der vorangegangenen Ansprüche, worin die Ester-Gruppe eine substituierte oder unsubstituierte Alkoxycarbonyl-Gruppe mit 1 bis 10 Kohlenstoffatomen oder eine substituierte oder unsubstituierte Phenoxycarbonyl-Gruppe ist.

5. Verfahren nach einem der vorangegangenen Ansprüche, worin die substituierte Silyl-Gruppe eine Trimethylsilyl-, i-Propyldimethylsilyl- oder t-Butyldimethylsilyl-Gruppe ist.

6. Verfahren nach einem der vorangegangenen Ansprüche, worin die Substituenten an der aliphatischen Kohlenwasserstoff-Gruppe oder an der Phenyl-Gruppe unabhängig voneinander aus Hydroxyl-, geschützten Hydroxyl-, Halogen-, Formyl-, Ester-, Carbonyl-, Amid-, Carboxy-, Cyano-, p-Methylthio-, Phenyl-, Fluorphenyl-, Chlorphenyl-, Bromphenyl- und Iodphenyl-Gruppen ausgewählt sind.

7. Verfahren nach einem der vorangegangenen Ansprüche, worin das Alkin der Formel (1) 1 -Methoxycarbonyl-1-heptin, 1-Phenyl-1-butin, 1-Hydroxy-2-octin, 3-Hydroxy-4-decin, 1-Carboxy-1-heptin, 3-Acetoxy-4-decin, 2-Hydroxy-2-methyl-3-octin, 1-t-Butyl-dimethylsiloxy-2-octin, 1-Tetrahydropyranoxy-2-octin oder 1-Ethoxycarbonyl-3-hexin ist.

8. Verfahren nach einem der vorangegangenen Ansprüche, worin der Palladiumkatalysator ein Komplex von Palladium mit einer Wertigkeit von 0 oder + 2 ist.

9. Verfahren nach einem der Ansprüche 1 bis 7, bei dem der Palladiumkatalysator aus Pd(PPh₃)₄, Pd(Ph^{t}Bu₂)₂, Pd(P^{t}Bu₃)₂, Pd[P(c-C₆H₁₁)₃]₂, Pd[P(OPh)₃]₄, Pd[P(OEt)₃]₄, Pd(AsPh₃)₄, Pd(CO)(PPh₃)₃, Pd(PPh₃)₂(CH₂=CH₂), Pd(PPh₃)₂(CF₂=CF₂), Pd[P(c-C₆H₁₁)₃]₂(CH₂=CH₂), Pd[P(c-C₆H₁₁)₃]₂(CF₂=CF₂), Pd(PBu₃)₂(CH₂=CH₂), Pd(PPh₃)₂(CF₂=CF₂), Pd[P(OC₆H₄CH₃-o)]₂(CH₂=CH₂), Pd(PPh₃)₂(CH₃OOCCH=CHCOOCH₃), Pd[P(OPh)₃]₂(CH₃OOCCH=CHCOOCH₃), Pd(PPh₃)₂(NCCH=CHCN), Pd[P(OPh)₃]₂(NCCH=CHCN), Pd(PPh₂Me)₂(PhCH=CH₂), Pd(PPh₂Me)₂(CH₂=CHCOOMe), Pd(PPh₂Me)₂[CH₂=CH(CH₃)CN], Pd(PPh₂Me)₂[CH₂=CH(CH₃)COMe], Pd(1,5-C₈H₁₂)₂, Pd₂(DBA)₃, Pd₃(CO)₃(P^{t}Bu₃)3, Pd₃(2,6-Me₂C₆H₃NC)₆, PdCl(CH₃)(COD), Pd(CH₃)₂(PEt₃)₂, Pd(C₂H₅)₂(PMe₂Ph)₂, Pd(CH₃)₂(DPPE), Pd(CH₃)(PPh₃)₂, PdPh₂(PEt₃)₂, Pd(CH₂Ph)₂(DMPE), Pd(CH₂Ph)₂(PMe₃)₂, Pd(CH₃)Cl(PEt₃)₂, Pd(CH₃)I(PMe₃)₂, Pd(CH₃)Br(PEt₃)₂, PdI(Ph)(PMe₃)₂, Pd(COPh)Cl(PMe₃)₂, Pd(COMe)I(PMe₃Ph₂)₂, Pd(COMe)Cl(PPh₃)₂, Pd(COEt)Cl[P(OMe₃)]₂, PdI(Ph)(PMe₂Ph)₂, Pd(CH≡CH)₂ (PEt₃)₂, Pd(PhC≡C)Cl(PBu₃)₂, Pd(Me₃CCH₂)₂(BPY), Pd(C₅H₆)Ph(PEt₃), Pd(η-C₃H₅)(C₅Me₅), [Pd(η-C₃H₅)(COD)]BF₄, [PdCl(1,1-Me₂-C₃H₅)]₂, Pd₂(DBA)₃CHCl₃, Pd(DBA)₂, PdCl₂(MeCN)₂, PdCl₂(PhCN)₂, Li₂PdCl₄, Na₂PdCl₄, [Pd(MeCN)₄](BF₄)₂, [Pd(η-C₃H₅)Cl]₂, Pd(η-C₃H₅)₂ und Pd(OAc)₂, ausgewählt ist, worin
DBA Dibenzylidenaceton ist,
COD Cyclooctadien ist,
DPPE Diphenylphosphinethan ist,
DMPE Dimethylphosphinethan ist,
BPY Dipyridyl ist.

10. Verfahren nach Anspruch 9, bei dem der Palladiumkatalysator Pd₂(DBA)₃CHCl₃ ist.

11. Verfahren nach einem der vorangegangenen Ansprüche, worin das Amin aus jenen der folgenden allgemeinen Formeln (3) bis (5) ausgewählt ist; jegliches Phosphin die folgende allgemeine Formel (6) aufweist und jegliches Phosphit die folgende allgemeine Formel (7) aufweist: 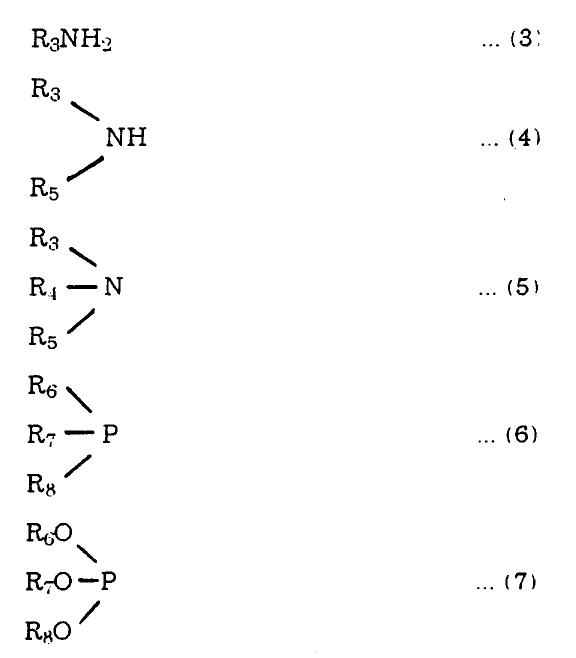 worin R₃, R₄, R₅, R₆, R₇ und R₈ unabhängig voneinander aus Kohlenwasserstoff-Gruppen mit 1 bis 10 Kohlenstoffatomen ausgewählt sind.

12. Verfahren nach Anspruch 1, bei dem ein Phosphin verwendet wird und n-Tributylphosphin ist und das Amin Triethylamin ist.

13. Verfahren nach einem der vorangegangenen Ansprüche, bei dem die Reduktionstemperatur im Bereich von -40°C bis zur Rückflußtemperatur des eingesetzten Lösungsmittels liegt und die Reaktionszeit ½ bis 12 h beträgt.

## Revendications

1. Méthode pour la préparation d'une cis-oléfine de formule générale (2) suivante: 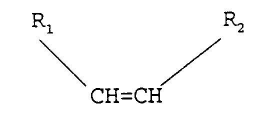 dans laquelle R₁ et R₂ sont indépendamment choisis parmi un groupe ester, un groupe silyle substitué, un groupe carboxyle, un groupe cyano, un groupe hydrocarboné aliphatique substitué ou non substitué ayant 1 à 20 atomes de carbone et un groupe phényle substitué ou non substitué, comprenant l'étape de réduction d'un alkyne de formule générale (1) suivante :
R₁ - C ≡ C - R₂ (1)
dans laquelle R₁ et R₂ sont tels que définis ci-dessus, avec de l'acide formique en présence d'un catalyseur au palladium, d'une amine et d'une phosphine ou d'un phosphite, l'amine étant ajoutée en une quantité allant de 0,1 à 10 équivalents par équivalent de l'alkyne et la phosphine ou le phosphite étant ajouté en une quantité allant de 0,1 à 100% en mol par rapport au nombre de moles de l'alkyne, la réduction étant effectuée dans un solvant choisi parmi des solvants de type éther, hydrocarbure, ester, amide et sulfoxyde et des mélanges de ceux-ci.

2. Méthode selon la revendication 1 dans laquelle le solvant est choisi parmi l'éther diéthylique, l'éther di-n-propylique, l'éther di-i-propylique, le tétrahydrofurane, le 1,4-dioxane, le n-pentane, le n-hexane, le benzène, le toluène, le xylène, l'acétate de méthyle, l'acétate d'éthyle, l'acétate de n-propyle, l'acétate de i-propyle, le diméthylformamide, le diméthylacétamide et le diméthylsulfoxyde.

3. Méthode selon la revendication 1 ou la revendication 2, dans laquelle le catalyseur au palladium est utilisé en une quantité allant de 1 à 10% en mol par rapport au nombre de moles de l'alkyne de formule (1), l'acide formique est utilisé en quantité allant de 10 à 500 % mol par rapport au nombre de moles de l'alkyne, l'amine est ajoutée en une quantité allant de 0,5 à 5 équivalents par équivalent de l'alkyne, et la phosphine ou le phosphite est ajouté en une quantité allant de 1 à 30% en mol par rapport au nombre de moles de l'alkyne.

4. Méthode selon l'une quelconque des revendications précédentes, dans laquelle le groupe ester est un groupe alcoxycarbonyle substitué ou non substitué ayant 1 à 10 atomes de carbone ou un groupe phénoxycarbonyle substitué ou non substitué.

5. Méthode selon l'une quelconque des revendications précédentes dans laquelle le groupe silyle substitué est un groupe triméthylsilyle, i-propyldiméthylsilyle ou t-butyldiméthylsilyle.

6. Méthode selon l'une quelconque des revendications précédentes dans laquelle les substituants sur le groupe hydrocarboné aliphatique ou sur le groupe phényle sont choisis indépendamment parmi les groupes hydroxyle, hydroxyle protégé, halogène, formyle, ester, carbonyle, amide, carboxy, cyano, p-méthylthio, phényle, fluorophényle, chlorophényle, bromophényle, et iodophényle.

7. Méthode selon l'une quelconque des revendications précédentes, dans laquelle l'alkyne de formule (1) est le 1-méthoxycarbonyle-1-heptyne, le 1-phényl-1-butyne, le 1-hydroxy-2-octyne, le 3-hydroxy-4-décyne, le 1-carboxy-1-heptyne, le 3-acétoxy-4-décyne, le 2-hydroxy-2-méthyl-3-octyne, le 1-t-butyldiméthylsiloxy-2-octyne, le 1-tétrahydropyranoxy-2-octyne ou le 1-éthoxycarbonyle-3-hexyne.

8. Méthode selon l'une quelconque des revendications précédentes, dans laquelle le catalyseur au palladium est un complexe de palladium avec une valence de zéro ou plus deux.

9. Méthode selon l'une quelconque des revendications 1 à 7, dans laquelle le catalyseur au palladium est choisi parmi Pd(PPh₃)₄, Pd(Ph^{t}Bu₂)₂, Pd(P^{t}Bu₃)₂, Pd[P(c-C₆H₁₁)₃]₂, Pd[P(OPh)₃]₄, Pd[P(OEt)₃]₄, Pd(AsPh₃)₄, Pd(CO) (PPh₃)₃, Pd(PPh₃)₂(CH₂=CH₂), Pd(PPh₃)₂(CF₂=CF₂), Pd[P(c-C₆H₁₁)₃]₂(CH₂=CH₂), Pd[P(c-C₆H₁₁)₃]₂(CF₂=CF₂), Pd(PBu₃)₂(CH₂=CH₂), Pd(PPh₃)₂(CF₂=CF₂), Pd[P(OC₆H₄CH₃)-o)]₂(CH₂=CH₂), Pd(PPh₃)2(CH₃OOCCH=CHCOOCH₃), Pd[P(OPh)₃]₂(CH₃OOCCH=CHCOOCH₃), Pd(PPh₃)₂(NCCH=CHCN), Pd[P(OPh)₃]₂(NCCH=CHCN), Pd(PPh₂Me)₂(PhCH=CH₂), Pd(PPh₂Me)₂(CH₂=CHCOOMe), Pd(PPh₂Me)₂[CH₂=CH(CH₃)CN], Pd(PPh₂Me)₂[CH₂=CH(CH₃)COMe], Pd(1,5-C₈H₁₂)₂, Pd₂(DBA)₃, Pd₃(CO)₃(P^{t}Bu₃)₃, Pd₃(2,6-Me₂C₆H₃NC)₆, PdCl(CH₃)(COD), Pd(CH₃)₂(PEt₃)₂, Pd(C₂H₅)₂(PMe₂Ph)₂, Pd(CH₃)₂(DPPE), Pd(CH₃)(PPh₃)₂, PdPh₂(PEt₃)₂, Pd(CH₂Ph)₂(DMPE), Pd(CH₂Ph)₂(PMe₃)₂, Pd(CH₃)Cl(PEt₃)₂, Pd(CH₃)I(PMe₃)₂, Pd(CH₃)Br(PEt₃)₂, PdI(Ph)(PMe₃)₂, Pd(COPh)Cl(PMe₃)₂, Pd(COMe)I(PMe₃Ph₂)₂, Pd(COMe)Cl(PPh₃)₂, Pd(COEt)Cl[P(OMe₃)]₂, PdI(Ph)(PMe₂Ph)₂, Pd(CH≡CH)₂, (PEt₃)₂, Pd(PhC≡C)Cl(PBu₃)₂, Pd(MeCCH₂)₂(BPY), Pd(C₅H₆)Ph(PEt₃), Pd(η-C₃H₅) (C₅Me₅), [Pd(η-C₃H₅)(COD)]BF₄, [PdCl(1,1-Me₂-C₃H₅]₂, Pd₂(DBA)₃CHCl₃, Pd(DBA)₂, PdCl2(MeCN)₂, PdCl₂(PhCN)₂, Li₂PdCl₄, Na₂PdCl₄, [Pd(MeCN)₄](BF₄)₂, [Pd(η-C₃H₅)Cl]₂, Pd(η-C₃H₅)₂ et Pd(OAc)₂. dans lesquels
DBA représente la dibenzylidèneacétone,
COD représente le cyclooctadiène,
DPPE répresente le diphénylphosphinéthane,
DMPE représente le diméthylphosphinéthane,
BPY représente le dipyridyle.

10. Méthode selon la revendication 9, dans laquelle le catalyseur au palladium est Pd₂(DBA)₃CHCl₃.

11. Méthode selon l'une quelconque des revendications précédentes, dans laquelle l'amine est choisie parmi celles de formule générale (3) à (5) suivante ; toute phosphine est l'une de celles qui ont la formule générale (6) suivante, et tout phosphite est l'un de ceux qui ont la formule générale (7) suivante. 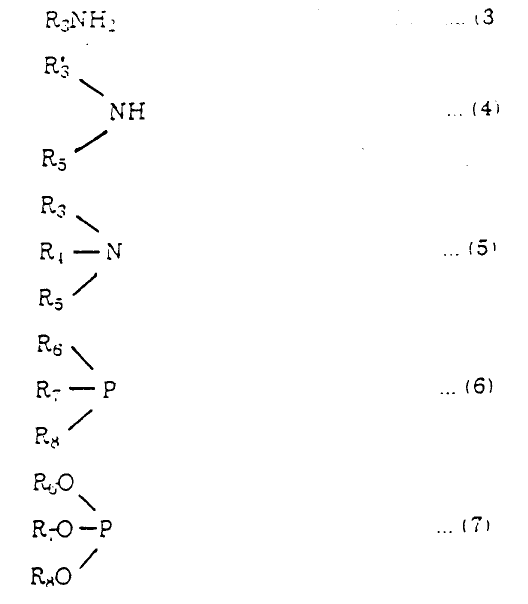 où, R₃, R₄, R₅, R₆, R₇ et R₈ sont indépendamment choisis parmi des groupes hydrocarbonés ayant 1 à 10 atomes de carbone.

12. Méthode selon la revendication 1, dans laquelle une phosphine est utilisée et est la n-tributylphosphine et l'amine est la triéthylamine.

13. Méthode selon l'une quelconque des revendications précédentes, dans laquelle la température de réduction est comprise dans l'intervalle allant de -40°C à la température de reflux du solvant utilisé et la durée de réaction est de 1/2 à 12 heures.
